(19) Europäisches Patentamt / European Patent Office / Office européen des brevets

(11) **EP 3 295 153 B1**

(12) **FASCICULE DE BREVET EUROPEEN**

(45) Date de publication et mention de la délivrance du brevet:
**28.06.2023 Bulletin 2023/26**

(21) Numéro de dépôt: **16729017.0**

(22) Date de dépôt: **11.05.2016**

(51) Classification Internationale des Brevets (IPC):
**G01N 21/27** (2006.01)   **A61B 1/04** (2006.01)
**A61B 5/00** (2006.01)   **G01N 21/64** (2006.01)

(52) Classification Coopérative des Brevets (CPC):
**G01N 21/6456; A61B 5/0071; A61B 5/6886; G01N 21/276;** A61B 1/043

(86) Numéro de dépôt international:
**PCT/FR2016/051114**

(87) Numéro de publication internationale:
**WO 2016/181076 (17.11.2016 Gazette 2016/46)**

(54) **PROCÉDÉ DE CORRECTION D'UNE IMAGE DE FLUORESCENCE**

VERFAHREN ZUR KORREKTUR EINES FLUORESZENZBILDES

METHOD OF CORRECTING A FLUORESCENCE IMAGE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **12.05.2015   FR 1554260**

(43) Date de publication de la demande:
**21.03.2018 Bulletin 2018/12**

(73) Titulaires:
• **Commissariat à l'énergie atomique et aux énergies alternatives**
**75015 Paris (FR)**
• **Fluoptics**
**38040 Grenoble (FR)**

(72) Inventeurs:
• **RIZO, Philippe**
**38700 La Tronche (FR)**
• **DAURES, Anthony**
**38000 Grenoble (FR)**

(74) Mandataire: **INNOV-GROUP**
**310, avenue Berthelot**
**69372 Lyon Cedex 08 (FR)**

(56) Documents cités:
EP-A1- 2 412 296     EP-A1- 2 505 988
EP-A2- 1 829 473     US-A- 5 749 830
US-A1- 2013 113 907     US-B1- 6 280 378

**Description**

## DOMAINE TECHNIQUE

Le domaine de l'invention est l'imagerie, et en particulier l'imagerie de fluorescence peropératoire pour le diagnostic et le suivi de l'évolution de pathologies ou de traitements dans le domaine médical.

## ART ANTERIEUR

L'imagerie de fluorescence est une technique permettant de localiser des marqueurs fluorescents dans un corps humain ou animal. Une des principales applications est la localisation de marqueurs fluorescents, ou fluorophores, ces derniers ciblant des cellules d'intérêt, par exemple des cellules cancéreuses. Le protocole consiste à injecter ces marqueurs dans l'organisme avant une intervention chirurgicale, de telle sorte qu'au cours de l'intervention, le praticien soit en mesure de visualiser les cellules cancéreuses à l'aide d'une l'image de fluorescence. Parce qu'elle permet d'acquérir une image indiquant la localisation des différentes zones cancéreuses, l'imagerie de fluorescence peropératoire permet d'obtenir une information jusqu'alors inaccessible au praticien, et constitue un complément utile, voire une alternative, à l'utilisation de traceurs radioactifs. Une autre application est l'assistance à des interventions, en chirurgie plastique et reconstructive, en chirurgie cardiovasculaire, en chirurgie du système lymphatique, ou en chirurgie hépatique, où l'imagerie de fluorescence permet le contrôle visuel du drainage lymphatique, de la perfusion ou de la vascularisation.

Le principe de l'imagerie de fluorescence est d'illuminer un champ d'observation à l'aide d'une source de lumière dans une bande spectrale d'excitation des fluorophores. Sous l'effet de cette illumination, les fluorophores émettent un rayonnement de fluorescence, dans une bande spectrale de fluorescence. Ce rayonnement peut être capté par une sonde de fluorescence, de manière à former une image de fluorescence sur laquelle apparaissent les différentes zones fluorescentes. Il est alors possible d'acquérir une image visible du champ observé, et de superposer l'image de fluorescence sur cette image visible.

La sonde de fluorescence peut être un ensemble compact, incluant la source de lumière d'excitation, et être portée par un praticien, en étant placée à quelques dizaines de centimètres d'un tissu examiné. Cependant, selon la position de la sonde par rapport au tissu, l'intensité des images de fluorescence peut varier, en particulier en fonction de la distance entre la sonde et le tissu observé. Aussi, si les images permettent de localiser correctement les zones fluorescentes, elles sont difficilement comparables, d'un point de vue quantitatif, les unes avec les autres, sauf à imposer au praticien de placer la sonde de fluorescence à une distance fixe par rapport au tissu observé. Certains dispositifs sont basés sur cette contrainte et ne permettent une approche quantitative qu'au prix du maintien de la sonde à une distance de consigne du tissu observé. Cela constitue un inconvénient important.

Le document US8606350 vise à résoudre ce problème, en utilisant une sonde de fluorescence apte à mesurer la distance la séparant d'un tissu biologique examiné. Il est alors possible de corriger l'image de fluorescence fournie par cette sonde en utilisant cette distance. L'objectif est d'aboutir à une imagerie de fluorescence quantitative, permettant notamment de quantifier et de comparer les niveaux d'intensité des zones fluorescentes observées. Pour cela, on acquiert une image de fluorescence, et on effectue une correction de cette image en la multipliant par la distance préalablement mesurée. Mais cette approche est basée sur l'hypothèse d'une relation inversement proportionnelle entre l'intensité du signal mesuré et la distance, approche qui ne permet pas de s'affranchir totalement de l'influence de la distance sur l'image de fluorescence obtenue. Le document EP1829473 suit une approche similaire, en décrivant un endoscope de fluorescence comportant une unité de mesure de distance entre l'endoscope et la surface d'un échantillon observé. La distance mesurée à l'aide de l'unité de mesure de la distance est utilisée pour corriger une image de fluorescence acquise par l'endoscope de fluorescence, en particulier en divisant ladite image de fluorescence par le carré de la distance mesurée.

Le document EP2505988 décrit un dispositif d'analyse de la fluorescence d'un échantillon. Ce dispositif permet d'acquérir une image d'un rayonnement de fluorescence émis par l'échantillon, cette dernière étant corrigée à l'aide d'une image de référence, obtenue en remplaçant l'échantillon par un échantillon de référence, ce dernier étant par exemple une plaque en acrylique.

Le document US2013/113907 décrit un endoscope de fluorescence, dont chaque image acquise est divisée par une image de référence, obtenue à l'aide d'un échantillon de référence, pour corriger les effets de vignettage de lentilles de l'endoscope. Une approche similaire est décrite dans US5749830.

Le document US6280378 décrit un endoscope de fluorescence, dans lequel l'image de fluorescence d'un échantillon est normalisée par une image de l'échantillon, ce dernier étant illuminé par une lumière à la longueur d'onde de fluorescence. L'efficacité d'un tel procédé dépend des propriétés optiques de l'échantillon, et nécessite une illumination de ce dernier par une lumière à la longueur d'onde de fluorescence. De façon similaire, le document EP2412296 décrit une normalisation d'une image de fluorescence d'un échantillon par une image visible de ce même échantillon. La correction effectuée dépend donc des propriétés de réflexion et de rétrodiffusion de l'échantillon, ce qui peut perturber l'interprétation des résultats.

Les inventeurs proposent d'améliorer les procédés décrits dans les documents préalablement cités, en proposant un procédé de correction amélioré d'une image de fluorescence d'un échantillon, indépendant des propriétés optiques de l'échantillon observé.

## EXPOSE DE L'INVENTION

Un objet de l'invention est un procédé de correction d'une image de fluorescence selon la revendication 1., Le procédé de l'invention comporte également une étape de mesure d'une distance entre la source d'excitation et l'objet, la fonction de correction étant dépendante de cette distance, en utilisant une image d'éclairement associée à ladite distance mesurée. La fonction de correction est telle que deux images d'éclairement, respectivement associées à deux distances mesurées différentes, sont différentes l'une de l'autre.

Le procédé peut comporter les caractéristiques suivantes, prises isolément ou selon les combinaisons techniquement réalisables :

- La fonction de correction peut effectuer un ratio, terme à terme, de l'image de fluorescence par ladite image d'éclairement.
- L'image d'éclairement peut être normalisée, en particulier par un niveau d'intensité maximal de cette image d'éclairement.
- L'image de fluorescence est acquise selon un temps d'exposition et la fonction de correction est également apte à normaliser l'image de fluorescence par rapport à un temps d'exposition de référence.
- La fonction de correction prend en compte le carré de la distance mesurée ainsi que le carré d'une distance de référence.
- Le procédé comporte une étape de sélection de la distance de référence en fonction de l'image de fluorescence.
- La fonction de correction comporte un produit de l'image de fluorescence par un ratio entre le carré de la distance mesurée et le carré de la distance de référence.
- La distance entre la source de lumière d'excitation et l'objet est mesurée au moyen d'un télémètre apte à émettre une onde en direction de l'objet et à détecter une onde réfléchie par l'objet, en fonction de laquelle ladite distance est estimée. Le télémètre peut alors être un télémètre laser, un télémètre à ultrasons ou un télémètre à micro-ondes.
- Le procédé comporte une étape de sélection de la distance de référence en fonction de l'image de fluorescence.
- La fonction de correction comporte un produit de l'image de fluorescence par un ratio entre le carré de la distance mesurée et le carré de la distance de référence.
- La distance entre la source de lumière d'excitation et l'objet est mesurée au moyen d'un télémètre apte à émettre une onde en direction de l'objet et à détecter une onde réfléchie par l'objet, en fonction de laquelle ladite distance est estimée. Le télémètre peut alors être un télémètre laser, un télémètre à ultrasons ou un télémètre à micro-ondes.
- La distance entre la source de lumière d'excitation et l'objet est mesurée par un télémètre optique comportant un photodétecteur matriciel, apte à déterminer une pluralité de distances entre respectivement la source de lumière d'excitation et une pluralité d'éléments de surface formant une surface de l'objet. Dans ce cas, la fonction de correction peut comporter un produit, terme à terme, de l'image de fluorescence par un ratio entre le carré de la distance mesurée entre la source d'excitation et un élément de surface et le carré d'une distance de référence.
- La distance entre la source de lumière d'excitation et l'objet est mesurée par un système autofocus, apte à établir automatiquement une distance de mise au point du capteur d'image de fluorescence par rapport à l'objet.
- Le procédé comporte une étape d'acquisition d'une image visible de l'objet à l'aide d'un capteur d'image visible.

Un autre objet de l'invention est un dispositif d'acquisition d'une image de fluorescence selon la revendication 11, comportant :

- une source de lumière d'excitation, apte à illuminer un objet dans une bande spectrale d'excitation,
- un capteur d'image de fluorescence, apte à collecter un rayonnement de fluorescence émis par ledit objet, dans une bande spectrale de fluorescence, sous l'effet de l'illumination par la source de lumière d'excitation, le capteur d'image de fluorescence étant apte à acquérir une image de fluorescence à partir du rayonnement de fluorescence collecté,
- un télémètre pour mesurer une distance entre la source de lumière d'excitation et l'objet, le télémètre étant apte à émettre une onde optique en direction de l'objet et à détecter une onde optique réfléchie par ledit objet, en fonction de laquelle ladite distance est mesurée,
- un processeur, apte à mettre en oeuvre le procédé de correction de ladite image de fluorescence tel que précédemment décrit.

Le dispositif d'acquisition, de préférence, comporte une ou plusieurs caractéristiques suivantes, prises isolément ou en combinaison :

- le capteur d'image de fluorescence est centré autour d'un axe optique et le télémètre est apte à émettre ladite onde optique selon ledit axe optique ;
- le capteur d'image de fluorescence comporte un système optique de focalisation et le télémètre est configuré pour émettre une onde optique, en direction de l'objet, centrée selon ce système optique ;
- le dispositif comporte un sélecteur d'une distance de référence, pour mémoriser ladite distance de référence dans une mémoire ;
- le télémètre comporte un photodétecteur matriciel, apte à déterminer une pluralité de distances respectivement entre la source d'excitation et une pluralité d'éléments de surface formant la surface de l'objet.

## FIGURES

La figure 1 représente un dispositif selon un premier mode de réalisation de l'invention.

La figure 2 représente une variante de ce dispositif.

La figure 3A représente une image de l'éclairement lorsque la source d'éclairement est placée à une distance de 10 cm de l'écran. La figure 3B représente un profil de l'intensité des pixels de l'image 3A selon une ligne horizontale passant par le centre de cette image.

La figure 4A représente une image de l'éclairement lorsque la source d'éclairement est placée à une distance de 20 cm de l'écran. La figure 4B représente un profil de l'intensité des pixels de l'image 4A selon une ligne horizontale passant par le centre de cette image.

La figure 5A représente une image de l'éclairement lorsque la source d'éclairement est placée à une distance de 30 cm de l'écran. La figure 5B représente un profil de l'intensité des pixels de l'image 5A selon une ligne horizontale passant par le centre de cette image.

La figure 6A représente une image de fluorescence non corrigée. La figure 6B représente une image de fluorescence corrigée selon un premier mode de réalisation d'un procédé de correction selon l'invention. La figure 6C représente des profils de l'intensité des pixels des images 6A et 6B selon une ligne horizontale passant par le centre de cette image.

Les figures 7A, 7B, 7C et 7D sont des logigrammes présentant les étapes de procédés de correction respectivement selon un premier, deuxième, troisième et quatrième mode de réalisation.

Les figures 8A, 8B, 8C et 8D sont des images obtenues respectivement sans correction, et en mettant en oeuvre une fonction de correction selon un premier, troisième et quatrième mode de réalisation.

La figure 9 représente un deuxième mode de réalisation d'un dispositif selon l'invention.

La figure 10 représente un troisième mode de réalisation d'un dispositif selon l'invention.

La figure 11 représente un quatrième mode de réalisation d'un dispositif selon l'invention.

La figure 12 représente un cinquième mode de réalisation d'un dispositif selon l'invention.

La figure 13 représente un sixième mode de réalisation d'un dispositif selon l'invention.

## EXPOSE DE MODES DE REALISATION PARTICULIERS

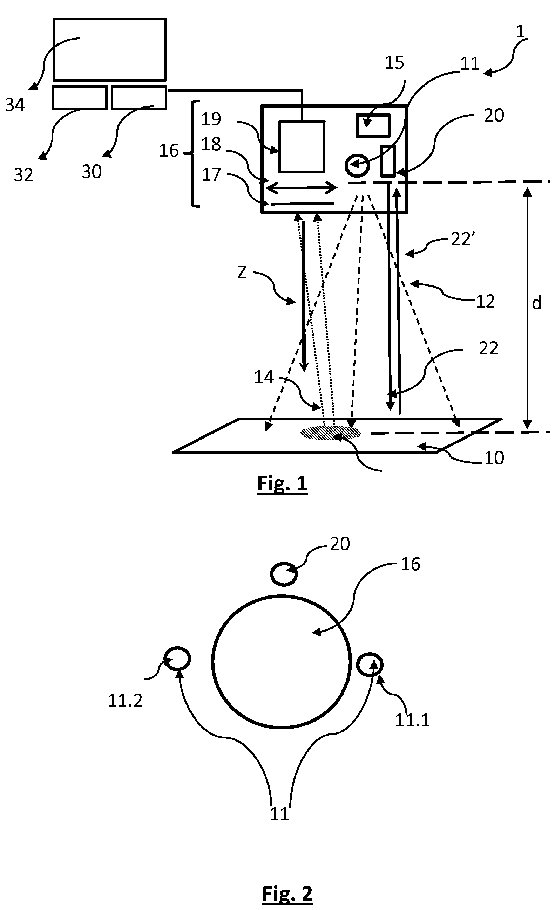

La figure 1 représente un premier mode de réalisation. Une sonde de fluorescence 1 comporte une source de lumière d'excitation 11, apte à émettre un faisceau lumineux d'excitation 12, dans une bande spectrale d'excitation $\lambda_{ex}$, de façon à illuminer un objet 10. L'objet 10 est un échantillon à caractériser, par exemple un tissu biologique, exposé à la sonde 1 au cours d'une intervention chirurgicale. La source de lumière est par exemple une diode électroluminescente, comprenant éventuellement un filtre d'excitation, apte à bloquer les longueurs d'onde en dehors de la bande spectrale d'excitation $\lambda_{ex}$. Il peut également s'agir d'une diode laser, ou d'une extrémité d'une fibre optique d'excitation, l'autre extrémité de cette fibre étant disposée face à une source lumineuse.

La source d'excitation 11 peut être divisée en plusieurs sources d'excitation élémentaires 11.1, 11.2...11.n.

La sonde comporte également un capteur d'image d'émission 16, comprenant un système optique de focalisation 18 et un photodétecteur matriciel 19. Le capteur d'image d'émission 16 est apte à former une image $I_{em}$ d'un rayonnement d'émission 14 produit par l'objet 10 sous l'effet de l'illumination par le faisceau d'excitation 12.

Ce rayonnement d'émission 14 peut être émis par l'objet dans la même bande spectrale que la bande spectrale d'excitation. Il s'agit alors d'un rayonnement réfléchi par l'objet, ou d'un rayonnement rétrodiffusé par l'objet.

Le rayonnement d'émission 14 peut être émis par l'objet dans une bande spectrale de fluorescence $\lambda_{fluo}$, différente de la bande spectrale d'excitation $\lambda_{ex}$. Il s'agit alors d'un rayonnement de fluorescence. Le capteur d'image d'émission est alors un capteur d'image de fluorescence, apte à former une image $I_{fluo}$ d'un rayonnement de fluorescence 14 produit par l'objet 10, dans ladite bande spectrale de fluorescence, sous l'effet de l'illumination par le faisceau d'excitation 12.

Dans les exemples qui suivent, on se place systématiquement dans le cas particulier de l'acquisition et de la correction d'une image de fluorescence, sachant que les principes décrits s'appliquent à tout type d'image $I_{em}$ émise par l'objet, quelle que soit la bande spectrale d'émission, et en particulier une image d'un rayonnement réfléchi par l'objet ou une image d'un rayonnement rétrodiffusé par l'objet.

Lorsque la sonde comporte plusieurs sources d'excitation élémentaires, ces dernières peuvent être réparties autour du capteur d'image de fluorescence 16.

Le capteur d'image de fluorescence 16 comporte de préférence un filtre 17. Le filtre 17 est un filtre passe-bande, centré dans la bande spectrale de fluorescence $\lambda_{fluo}$, de façon à bloquer les longueurs d'onde en dehors de cette bande spectrale de fluorescence.

Le système optique de focalisation 18 est configuré pour former une image $I_{fluo}$ du rayonnement de fluorescence 14 sur le photodétecteur matriciel 19. Il peut notamment s'agir d'un objectif. Le photodétecteur matriciel comporte plusieurs pixels, de telle sorte que chaque pixel de l'image de fluorescence $I_{fluo}$ est optiquement couplé, par le système optique de focalisation 18, à un élément de surface $\delta S$ de l'objet 10, un tel élément de surface formant une partie d'une surface S de l'objet 10 placée face à la sonde 1.

Le capteur de fluorescence 16 comporte un axe optique Z, défini par le système optique de focalisation 18 et le photodétecteur 19.

Le photodétecteur matriciel 19 est un capteur de type CCD (acronyme anglais signifiant Charge Coupled Device - Dispositif à Transfert de Charge) ou un capteur de type CMOS (acronyme anglais signifiant Complementary Metal-Oxyde Semiconductor).

L'objet 10 comporte un ou plusieurs fluorophores endogènes ou exogènes. Dans le cas où les fluorophores sont endogènes, on parle d'autofluorescence. Les fluorophores exogènes sont préalablement injectés dans l'objet, de manière à se fixer spécifiquement sur des cibles, par exemple des cellules cancéreuses. Chaque fluorophore est apte à émettre un rayonnement de fluorescence 14, dans une bande spectrale de fluorescence $\lambda_{fluo}$, lorsqu'il est illuminé par un rayonnement d'excitation 12, dans une bande spectrale d'excitation $\lambda_{ex}$. Par exemple, lorsque le fluorophore utilisé est du vert d'indocyanine, ou ICG (acronyme anglais signifiant IndoCyanine Green), la bande spectrale d'excitation peut être comprise entre 750 nm et 800 nm, la bande spectrale de fluorescence étant comprise entre 820 nm et 870 nm.

L'image de fluorescence $I_{fluo}$ comporte des pixels r. La valeur de chaque pixel $I_{fluo}(r)$ correspond à l'intensité du rayonnement de fluorescence émanant d'un élément de surface $\delta S(r')$ de l'objet, cet élément de surface optiquement couplé à ce pixel. Les positions r et r' désignent respectivement des coordonnées d'un pixel dans l'image de fluorescence $I_{fluo}$ et d'un élément de surface sur la surface S de l'objet 10. Chaque position r et/ou r' peut correspondre à une abscisse x et une ordonnée y, de telle sorte que r = (x,y) et/ou r' = (x', y').

L'intensité du rayonnement de fluorescence émanant de chaque élément de surface peut également être qualifiée d'intensité de fluorescence apparente, la fluorescence pouvant être produite à la surface de l'objet ou en profondeur, cette profondeur étant généralement limitée à quelques centimètres. L'image de fluorescence $I_{fluo}$ générée par le capteur d'image 16 donne l'apparence que la fluorescence provient de la surface S de l'objet 10, alors qu'elle peut être générée en profondeur.

Un processeur 30 est apte à traiter les images de fluorescence $I_{fluo}$ formées par le capteur d'image 16. Il s'agit par exemple d'un microprocesseur placé dans un ordinateur. En particulier, le processeur est un microprocesseur 30 relié à une mémoire programmable 32 dans laquelle est stockée une séquence d'instructions pour effectuer les opérations de traitement d'images et de calculs décrites dans cette description. Le microprocesseur 30 peut être relié à un écran d'affichage 34.

Lorsque l'objet comporte une zone fluorescente 13, située dans le champ d'observation de la sonde 1, cette zone fluorescente apparaît, sur l'image de fluorescence $I_{fluo}$, sous la forme d'une région d'intérêt $\Omega$. La région d'intérêt $\Omega$ peut être caractérisée par un signal de fluorescence $S_{fluo}$, représentatif de l'intensité des pixels qui la compose.

La sonde 1 comporte également un détecteur de distance, sous la forme d'un télémètre 20, destiné à mesurer une distance d entre la source lumineuse d'excitation 11 et l'objet 10. Ce télémètre 20 peut être un télémètre optique, à ultrasons ou à micro-ondes. D'une façon générale, le télémètre 20 émet une onde, dite onde de télémétrie 22 vers l'objet 10, puis détecte une onde 22' réfléchie par l'objet, à partir de laquelle une mesure de la distance est effectuée. L'onde 22 peut être une onde optique dans le spectre visible ou infrarouge, ou une onde acoustique ou encore une onde électromagnétique de type micro-onde. Dans cet exemple, le télémètre 20 est un télémètre laser. Le télémètre peut notamment être un télémètre à temps de vol, apte à déterminer une durée entre l'émission de l'onde de télémétrie et la détection de l'onde de télémétrie réfléchie par l'objet.

Dans les exemples représentés sur les figures 1 et 2, le capteur d'image 16, la source de lumière d'excitation 11 ainsi que le télémètre 20 sont intégrés dans la sonde 1.

La sonde 1 est généralement placée à une distance de l'objet 10 comprise entre 10 cm et 30 voire 50 cm ou davantage. Elle peut être déplacée manuellement par un praticien, de façon à localiser d'éventuelles zones fluorescentes 13 dans l'objet 10. Ces zones fluorescentes peuvent être situées en surface ou à quelques millimètres, voire centimètres, de profondeur. Le praticien peut par exemple porter une telle sonde dans sa main, et balayer le champ opératoire afin de réaliser un examen complet.

L'éclairement $\phi$ produit par la source de lumière d'excitation 11 sur l'objet examiné est souvent spatialement inhomogène, et comporte des zones plus éclairées que d'autres, définissant une image d'éclairement. On rappelle que l'éclairement $\phi$ correspond à une quantité de lumière par unité de temps et de surface. L'image d'éclairement dépend généralement de la distance d entre la source de lumière 11 et l'objet 10 et peut varier significativement entre deux distances différentes.

La figure 2 représente une vue de face d'une variante selon laquelle la source d'excitation 11 comporte deux sources d'excitation élémentaires 11.1 et 11.2 disposées de part et d'autre du capteur de fluorescence 16. Chacune de ces sources d'excitation élémentaire comporte une pluralité de fibres optiques, des extrémités desquelles est émis le faisceau d'excitation 12.

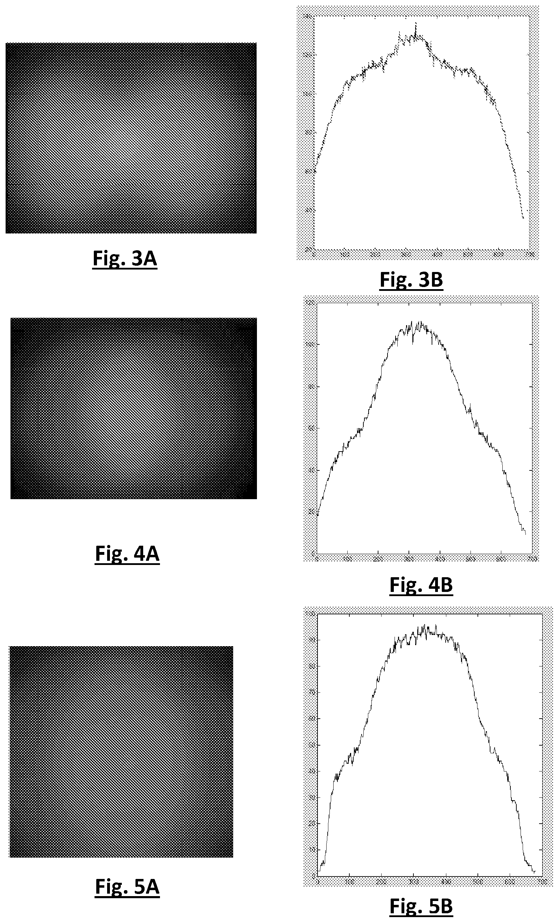

Les figures 3A, 4A et 5A représentent, pour différentes distances d, la distribution spatiale de l'éclairement, dans un plan perpendiculaire à l'axe de propagation du faisceau d'excitation 12, avec une telle configuration. Les deux sources d'excitation 11.1 et 11.2 sont constituées par l'extrémité de deux fibres optiques reliées à une source laser émettant à la longueur d'onde d'excitation de 750 nm.

Pour réaliser ces figures, on a disposé un écran fluorescent homogène face à la sonde, la distance entre l'écran et la sonde étant respectivement de 10 cm, 20 cm et 30 cm. Les figures 3B, 4B et 5B représentent le profil horizontal, passant par le centre de l'image, pour chaque configuration.

Les inventeurs ont constaté que, quelle que soit la distance, l'éclairement n'est pas homogène. Il est maximal au centre du champ observé, et décroît significativement au fur et à mesure qu'on se rapproche des bords de l'image. Par ailleurs, les inventeurs ont observé que la distribution spatiale de l'éclairement varie en fonction de la distance, en particulier lors des applications peropératoires, où la distance entre la sonde et la scène observée peut varier entre quelques centimètres et quelques dizaines de centimètres.

Cette hétérogénéité spatiale nuit à une quantification correcte des signaux de fluorescence $S_{fluo}$, l'intensité du rayonnement de fluorescence 14 variant de façon linéaire avec l'éclairement $\phi$ produit par le faisceau d'excitation 12. Ainsi, avec un tel éclairement spatialement inhomogène, le rapport signal sur bruit de la sonde n'est pas homogène : il est nettement plus élevé dans la zone centrale du champ observé que dans la zone périphérique de ce champ.

Selon un premier mode de réalisation, afin de remédier à ce problème, les inventeurs proposent d'appliquer une fonction de correction $f_d$, dépendant de ladite distance d, à chaque image de fluorescence. Cette fonction corrige l'image de fluorescence $I_{fluo}$ de façon à obtenir une image de fluorescence $I'_{fluo}$ corrigée, telle que :

$$I'_{fluo} = f_d(I_{fluo}) = \frac{I_{fluo}}{M_d}, \qquad (1a)$$

$M_d$ correspondant à une image d'éclairement produite par la source de lumière 11 à la distance d. Cette image d'éclairement est représentative de la distribution spatiale de l'éclairement produit par la source d'excitation dans un plan situé à une distance d de cette dernière.

Le ratio $\frac{I_{fluo}}{M_d}$ sous-entend que l'intensité de chaque pixel de coordonnée (r) de l'image de fluorescence $I_{fluo}$ est divisée par l'intensité d'un pixel de même coordonnée de l'image d'éclairement $M_d$. Autrement dit, il s'agit d'un ratio entre deux matrices, terme à terme, de telle sorte que la valeur de chaque pixel r de l'image corrigée soit :

$$I'_{fluo}(r) = f_d(I_{fluo}(r)) = \frac{I_{fluo}(r)}{M_d(r)}$$

A chaque distance d est associée une image d'éclairement $M_d$, cette dernière étant déterminée lors d'une phase de calibration, en remplaçant l'objet 10 par un écran homogène, par exemple un écran noir, ou, mieux, un écran fluorescent, la fluorescence étant spatialement homogène. Un tel écran fluorescent peut être constitué d'un matériau naturellement fluorescent, dans la bande de longueur d'onde de fluorescence $\lambda_{fluo}$ du capteur d'image de fluorescence 16. Il peut par exemple s'agir d'un polymère, par exemple une plaque de polyméthacrylate de méthyle, matériau connu sous l'acronyme PMMA, commercialisée sous la référence Altuglas (marque déposée) 25001.

L'image formée sur cet écran, placé à une distance *d* de la source de lumière 11, correspond alors à l'image d'éclairement $M_d$ associée à ladite distance *d*. Elle comporte des zones claires et des zones sombres, représentant la répartition spatiale, souvent inhomogène, de l'éclairement $\phi$ en fonction de la distance $d$. Plusieurs images d'éclairement $M_{d1}...M_{dn}$ peuvent être réalisées en considérant différentes distances $d_1...d_{n'}$ , par exemple tous les 1 ou 2 cm. Des images correspondant à des distances intercalaires, entre deux distances $d_i$, $d_{i+1}$, peuvent être obtenues par interpolation entre deux images $M_{di}$ et $M_{di+1}$. On mémorise alors autant d'images d'éclairement $M_d$ que de distances $d$ considérées. Les images d'éclairement $M_{d'}$ et $M_{d''}$, respectivement associées à deux distances différentes $d'$et $d''$, sont généralement différentes. Les différentes images $M_d$, correspondant à chaque distance considérée, peuvent être mémorisées dans une mémoire 32 reliée au processeur 30.

Une image $M_d$ d'éclairement peut également être réalisée pour différentes configurations du système optique de focalisation 18 utilisé dans le capteur d'image de fluorescence 16, en particulier en fonction d'un grandissement G de ce système optique, de façon à constituer des images d'éclairement $M_{d,G}$ correspondant à différents couples de valeurs de la distance $d$, et du grandissement G.

Chaque image d'éclairement $M_d$ ou $M_{d,G}$ permet de prendre en compte le vignettage affectant le capteur d'image de fluorescence 16, ou, de façon plus générale, toute inhomogénéité dans la fonction de réponse de ce capteur, qu'elle soit due à l'objectif 18 ou au photodétecteur matriciel 19, voire au filtre 17. Dans ces conditions, chaque image d'éclairement $M_d$ ou $M_{d,G}$ peut être qualifiée d'image de blanc, car elle correspond à une image de calibration, sur un fond homogène, de la sonde lorsque la source de lumière 11 est activée.

Chaque zone fluorescente 13 de l'objet peut alors être caractérisée non seulement pas sa position et son étendue spatiale, telle qu'elle apparaît sur la région d'intérêt $\Omega$ de l'image de fluorescence, mais également par une information quantitative, sous la forme d'un signal $S_{fluo}$ représentatif de l'intensité des pixels constituant cette région d'intérêt. Si $\Omega$ définit l'étendue spatiale de ladite région d'intérêt sur l'image de fluorescence $I'_{fluo}$, l'information quantitative correspond au signal de fluorescence $S_{fluo}$ . Ce dernier peut être défini, de manière non exhaustive, par l'une des expressions suivantes,

- l'intensité maximale dans la région d'intérêt $\Omega : S_{fluo} = \max_{\Omega}(i'_{fluo}(r))$ ;

- l'intensité moyenne dans la région d'intérêt $\Omega: S_{fluo} = \underset{\Omega}{\mathrm{mean}}(i'_{fluo}(r))$ , où mean désigne l'opérateur moyenne ;

- l'intensité totale dans la région d'intérêt $\Omega : S_{fluo} = \int_{\Omega} i'_{fluo}(r)$ ;

$I_{fluo}(r)$ désigne l'intensité d'un pixel de coordonnée r dans l'image de fluorescence corrigée $I'_{fluo}$.

Le praticien est alors en mesure de comparer les différentes zones fluorescences non seulement en fonction de l'étendue des zones d'intérêts sur l'image de fluorescence, mais également en fonction de leurs intensités respectives, la fonction de correction $f_d$ ayant corrigé l'hétérogénéité spatiale de l'éclairement. La correction peut être réalisée en temps réel, c'est-à-dire à la cadence d'acquisition des images de fluorescence, ou en post-traitement.

Le recours à une telle image d'éclairement $M_d$ présente l'avantage d'une prise en compte, lors de l'application de la fonction de correction, de la distribution spatiale de l'éclairement, mais également de l'évolution de l'éclairement en fonction de la distance d entre la source d'excitation et l'objet. Or, l'intensité de la lumière de fluorescence 12 émise par l'objet 10 est proportionnelle à l'éclairement. Ainsi, en mettant en oeuvre une fonction de correction prenant en compte une image d'éclairement, le procédé permet de corriger à la fois l'inhomogénéité spatiale de l'éclairement ainsi que l'évolution de ce dernier, en fonction de la distance entre la source de lumière d'excitation 11 et l'objet 10.

Une telle correction permet d'obtenir une image de fluorescence corrigée $I'_{fluo}$ dans laquelle la valeur de chaque pixel $I'_{fluo}(r)$ est représentative d'une concentration de fluorophores apparente, dans un élément de surface de l'objet $\delta S(r')$ optiquement couple audit pixel r. En effet, comme précédemment indiqué, $I_{fluo}(r)$ correspond à l'intensité de la lumière fluorescence émanant de l'élément de surface $\delta S(r')$ conjugué du pixel r de l'image $I_{fluo}$. Ainsi,

$$I_{fluo}(r) \propto \phi_d(r') \times \eta \times c(r') \quad (1')$$

où :

- $\phi_d(r')$ désigne l'éclairement que subit l'élément de surface $\delta S(r')$ de l'objet, se trouvant à la distance d de la source d'excitation ;
- $\eta$ désigne le rendement quantique du fluorophore générant le signal de fluorescence ;
- c(r') désigne la concentration apparente du fluorophore à l'élément de surface $\delta S(r')$.

Le terme concentration apparente désigne une concentration de fluorophore à l'origine du signal de fluorescence émanant de l'élément de surface $\delta S(r')$.

Lors de l'acquisition de l'image d'éclairement $M_d$, à l'aide d'un écran fluorescent, chaque pixel de l'image $M_d$

est tel que :

$$M_d(r) \propto \phi_d(r') \times \eta \times c_0(r') \quad (1'')$$

où

- $\phi_d(r')$ désigne l'éclairement sur l'écran fluorescent ;
- $\eta$ désigne le rendement quantique du fluorophore générant le signal de fluorescence ;
- $c_0(r')$ désigne la concentration apparente de fluorophore dans l'écran de calibration, au point r' conjugué du point r de l'image. Cette concentration est de préférence homogène dans l'écran, si bien que $c_0(r') = c_0$.

On note que la même source d'excitation étant utilisée lors des acquisitions respectives de l'image de fluorescence et de l'image d'éclairement. L'éclairement produit par la source d'excitation à la distance d, $\phi_d(r')$, est donc le même lors de l'acquisition de ces deux images.

L'image corrigée $I'_{fluo}$, prenant la forme d'un ratio, terme à terme, entre l'image de fluorescence $I_{fluo}$ et l'image d'éclairement $M_d$, est donc telle que :

$$I'_{fluo}(r) = f_d(I_{fluo}(r)) = \frac{I_{fluo}(r)}{M_d(r)} \propto \frac{c(r')}{c_0} \propto c(r') \quad (1''')$$

Ainsi, l'image corrigée représente la distribution spatiale de la concentration du fluorophore à la surface S de l'objet 10.

Le signal de fluorescence $S_{fluo}$, tel que précédemment défini, associé à une zone de fluorescence 13, représente alors, selon sa définition, la concentration totale ou moyenne du fluorophore dans la zone concernée.

Selon une variante, chaque image d'éclairement est normalisée. La normalisation peut être effectuée en prenant en compte la valeur des pixels dans lesquels l'éclairement est maximal. Ainsi, la normalisation peut consister à effectuer l'opération suivante :

$$M'_d(r) = \frac{M_d(r)}{\max(M_d(r))}$$

- $M'_d(r)$ représentant la valeur du pixel de coordonnée r de l'image d'éclairement normalisée $M'_d$;
- $M_d(r)$ représentant la valeur du pixel de coordonnée r de l'image d'éclairement $M_d$;
- $max(M_d(r))$ représentant la valeur du pixel de l'image d'éclairement $M_d$ représentatif de l'éclairement maximal.

Selon cette variante, la fonction de correction permet d'établir une image de fluorescence $I'_{fluo}$ corrigée, telle que :

$$I'_{fluo} = f_d(I_{fluo}) = \frac{I_{fluo}}{M'_d} \quad (1b)$$

Un avantage d'une telle normalisation qu'elle permet une correction indépendante de l'intensité de la source d'excitation, et de ses éventuelles fluctuations.

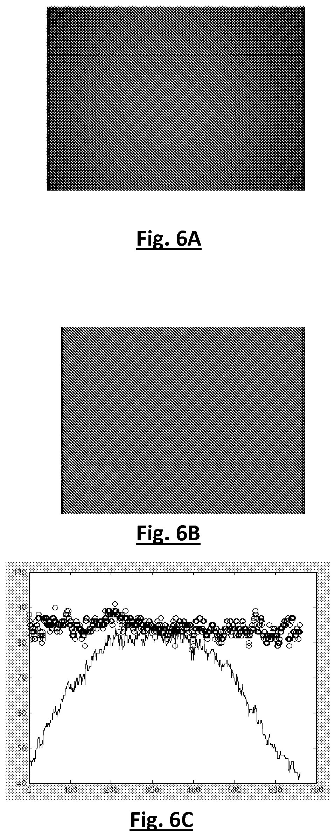

La figure 6A représente une image de fluorescence $I_{fluo}$ non corrigée, lorsque la distance entre la source et un écran homogène s'élève à 20 cm, en utilisant une image d'éclairement normalisée $M'_d$. La figure 6B représente une image de fluorescence $I'_{fluo}$ corrigée en mettant en oeuvre l'équation (1b). La figure 6C représente un profil horizontal, passant par le centre de l'image, pour chacune de ces images. On observe que le profil associé à l'image corrigée témoigne, aux fluctuations près, d'un signal de fluorescence homogène, correspondant à la réalité, ce qui n'est pas le cas sur l'image non corrigée.

Selon une variante non revendiquée, la fonction de correction est établie pour une distance à laquelle on considère que l'éclairement est suffisamment représentatif d'une certaine plage de distance. Selon cette variante, la fonction de correction f ne varie pas avec la distance, et peut utiliser la même image d'éclairement M, normalisée ou non, quelle que soit la distance utilisée. Dans ce cas :

$$I'_{fluo} = f(I_{fluo}) = \frac{I_{fluo}}{M}, \qquad (1c)$$

La mise en oeuvre des corrections, quelles qu'elles soient, décrites en lien avec ce premier mode de réalisation, suppose, de préférence, que la position relative du capteur d'image de fluorescence et de la source de lumière d'excitation soit connue et qu'elle corresponde aux conditions dans lesquelles les images d'éclairement $M_d$ sont établies. Aussi, une telle correction est particulièrement adaptée aux sondes intégrant, dans un même corps, la source de lumière ainsi que le capteur d'image de fluorescence, la position relative de la source de lumière et du capteur d'image de fluorescence étant fixe. Les dispositifs représentés sur les figures 1, 2, 9, 10 et 11 correspondent à ce cas de figure.

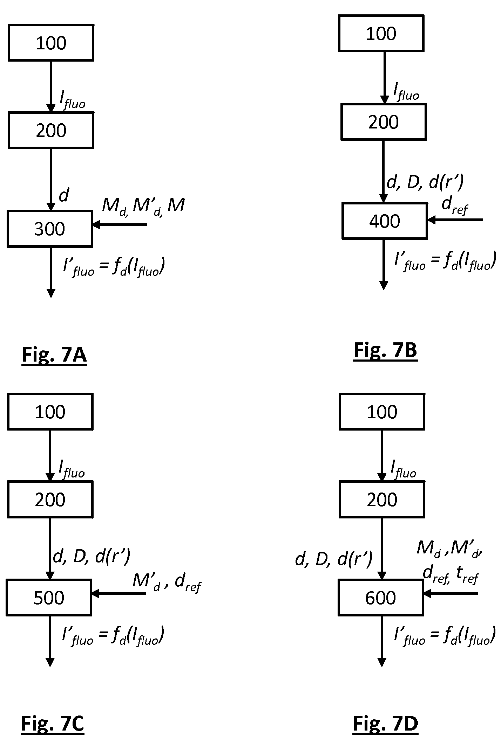

En lien avec la figure 7A, on décrit à présent les principales étapes d'un procédé de correction d'une image de fluorescence en fonction de la distance d entre la source de lumière d'excitation 11 et l'objet 10, selon ce premier mode de réalisation.

Au cours d'une étape 100, on acquiert une image de fluorescence $I_{fluo}$. Au cours d'une étape 200, on mesure la distance $d$ entre la source de lumière d'excitation 11 et l'objet 10, par exemple au moyen du télémètre 20 ou au moyen d'un système de mise au point automatique de type autofocus 27, décrit par la suite.

L'étape 300 consiste à appliquer une fonction de correction de distance $f_d$, dépendant de ladite distance $d$, à chaque image de fluorescence $I_{fluo}$, de façon à obtenir une image de fluorescence $I'_{fluo}$ corrigée, selon l'équation (1a), (1b) ou (1c).

Selon un deuxième mode de réalisation, non revendiqué, la fonction de correction a pour effet de compenser les variations de l'intensité d'éclairement en fonction de la distance entre l'objet 10 et la source de lumière 11.

La source de lumière 11 étant généralement placée à une distance supérieure à 10 cm de l'objet observé, elle peut être considérée, vue de l'objet, comme ponctuelle. Selon cette hypothèse, la quantité de lumière illuminant l'objet par unité de surface et de temps, c'est-à-dire l'éclairement $\phi$, varie de façon inversement proportionnelle au carré de la distance d entre la source de lumière 11 et l'objet 10, selon une loi dite en carré inverse de type $\frac{1}{4\pi d^2}$.

Or, la quantité de signal de fluorescence émis par une zone fluorescente 13 de l'objet est proportionnelle à la quantité de lumière d'excitation atteignant cette zone par unité de temps, c'est-à-dire à l'éclairement produit par la source de lumière d'excitation sur ladite zone. Lorsque le praticien éloigne ou rapproche la sonde 1 d'une même zone fluorescente 13, l'intensité du rayonnement de fluorescence émis par cette zone varie, et l'intensité du signal $S_{fluo}$ de la région d'intérêt $\Omega$ apparaissant sur l'image de fluorescence est modifiée en conséquence. Cette variation d'intensité est très marquée, du fait de sa dépendance selon l'inverse du carré de la distance. Par exemple, si la distance d passe de 20 cm à 40 cm, la variation d'intensité sur l'image de fluorescence peut atteindre un facteur 4.

Si $\phi$ et $\phi_{ref}$ correspondent respectivement à l'éclairement produit par la source de lumière d'excitation 11 à une distance d et à une distance de référence $d_{ref}$,

$$\frac{\phi}{\phi_{ref}} = \frac{d_{ref}^2}{d^2} \quad (2)$$

Aussi, si $I_{fluo\text{-}ref}(r)$ et $I_{fluo}(r)$ représentent l'intensité d'un rayonnement de fluorescence, provenant d'un même élément de surface $\delta S(r')$, collecté par un pixel de coordonnée r de l'image $I_{fluo}$, lorsque la source de lumière 11 est placée respectivement à la distance de référence $d_{ref}$ et à la distance d de l'objet,

$$I_{fluo}(r) = I_{fluo-ref}(r) \times \frac{d_{ref}^2}{d^2} \quad (3)$$

Selon ce mode de réalisation, l'effet de la fonction de correction $f_d$ est de compenser la variation de distance par rapport à la distance de référence $d_{ref}$. Aussi, si une image $I_{fluo}$ est acquise à une distance de fluorescence d, la fonction de correction a pour effet de ramener l'intensité $I_{fluo}(r)$ de chaque pixel, correspondant à une zone de fluorescence 13, à une valeur de référence $I_{fluo\text{-}ref}(r)$ correspondant à la distance de référence $d_{ref}$. Après correction, l'intensité corrigée $I'_{fluo}(r)$ au niveau du pixel r est alors :

$$I'_{fluo}(r) = f_d\left(I_{fluo}(r)\right) = I_{fluo}(r) \times \frac{d^2}{d_{ref}^2} \quad (4)$$

Compte tenu de l'équation (3), il vient :

$$I'_{fluo}(r) \approx I_{fluo-ref}(r) \quad (5),$$

le symbole $\approx$ représentant une égalité aux fluctuations statistiques près.

Il est possible d'appliquer ce raisonnement à l'ensemble des pixels constituant une image de fluorescente. Ainsi, si $I_{fluo}$ et $I'_{fluo}$ représentent une image de fluorescence respectivement avant et après application de la fonction de correction $f_d$,

$$I'_{fluo} = f_d(I_{fluo}) = I_{fluo} \times \frac{d^2}{d_{ref}^2} \quad (6a)$$

Chaque pixel de l'image $I_{fluo}$ est alors multiplié par le scalaire $\frac{d^2}{d_{ref}^2}$.

La distance de référence $d_{ref}$ peut être est une distance prédéterminée, par exemple 20 cm.

La distance de référence $d_{ref}$ peut également être déterminée au cas par cas par le praticien mettant en oeuvre la sonde 1. Le praticien dispose alors d'un sélecteur 15, par exemple un bouton poussoir, disposé sur la sonde 1, de telle sorte qu'en actionnant ledit sélecteur 15, la distance à laquelle se trouve la sonde par rapport à l'objet est considérée comme étant une distance de référence. Cette distance est alors mémorisée dans une mémoire 32 en tant que distance de référence pour les prochaines corrections à établir. Cela permet de sélectionner la distance de référence $d_{ref}$ au cas par cas, en fonction de l'image de fluorescence ($I_{fluo}$) correspondant à cette distance, cette image étant appelée image de fluorescence de référence ($I_{fluo-ref}$). Ce sélecteur 15 peut être prévu sur l'ensemble des modes de réalisation décrits.

L'équation (5) montre que l'application de la fonction de correction $f_d$ fait en sorte qu'une même zone fluorescente 13 produit, sur l'image de fluorescence corrigée $I'_{fluo}$, une région d'intérêt $\Omega$, dont l'intensité $i'_{fluo}(r)$ de chaque pixel est peu dépendante, voire indépendante, de la distance d entre la source de lumière d'excitation 11 et l'objet examiné 10.

Selon une variante le télémètre 20 est un télémètre optique met en oeuvre un photodétecteur pixelisé, permettant une mesure d'une pluralité de distances d(r') entre respectivement la source d'excitation et une pluralité d'éléments de surface $\delta S(r')$ de l'objet (10). Autrement dit, le capteur de télémétrie 20 comporte un photodétecteur matriciel, détectant l'onde 22' réfléchie par l'objet. Chaque pixel de ce photodétecteur est apte à établir une mesure de la distance le séparant de l'élément de surface de l'objet dont il est optiquement couplé, c'est-à-dire de l'élément de surface conjugué de ce pixel. Un tel photodétecteur peut par exemple être une caméra 3D à temps de vol, par exemple le modèle SR4000 commercialisé par la société Mesa Imaging.

Selon ce mode de réalisation, on peut associer, à chaque pixel r de l'image de fluorescence $I_{fluo}$, une distance d(r') entre la source d'excitation 11 et l'élément de surface $\delta S(r')$ conjugué du pixel r.

La correction peut donc être réalisée non pas en appliquant un terme de correction scalaire sur toute l'image $I_{fluo}$, mais, à l'instar du premier mode de réalisation, terme à terme, de telle sorte que

$$I'_{fluo}(r) = f_d(I_{fluo}(r)) = I_{fluo}(r) \times \frac{d(r')^2}{d_{ref}^2} \quad (6b)$$

Une autre façon d'exprimer cette correction est de considérer qu'à partir de chaque distance d(r') mesurée, on forme une image de distance D, chaque terme D(r) de cette image de distance correspondant à la distance entre l'élément de surface $\delta S(r')$ conjugué du pixel r de l'image $I_{fluo}$ et la source de lumière d'excitation. La correction comprend alors un produit terme à terme de l'image de fluorescence $I_{fluo}$ par le carré de l'image D, pondéré par le scalaire $\frac{1}{d_{ref}^2}$. Cela peut être exprimé comme suit :

$$I'_{fluo}(r) = f_d(I_{fluo}(r)) = I_{fluo}(r) \times \frac{D(r)^2}{d_{ref}^2} \quad (6c)$$

En lien avec la figure 7B, on décrit à présent les principales étapes d'un procédé de correction d'une image de fluorescence en fonction de la distance d entre la source de lumière d'excitation 11 et l'objet 10, selon ce deuxième mode de réalisation. Les étapes 100 et 200 sont similaires à celles décrites relativement à la figure 7A. L'étape 400 consiste à appliquer une fonction de correction de distance $f_d$, dépendant de ladite distance d, à chaque image de fluorescence $I_{fluo}$, de façon à obtenir une image de fluorescence $I'_{fluo}$ corrigée, selon l'équation (6a), (6b) ou (6c).

Les fonctions de correction décrites dans le premier et le deuxième mode de réalisation peuvent être mises en oeuvre indépendamment l'une de l'autre, mais également combinées.

Ainsi, selon un troisième mode de réalisation, la fonction de correction combine la correction de l'inhomogénéité spatiale de l'éclairement et la compensation de la distance. On applique à l'image une fonction de correction combinant les fonctions de correction décrites en lien avec le premier ou le deuxième mode de réalisation. Cette fonction $f_d$ peut-être telle que telle que :

$$I'_{fluo} = f_d(I_{fluo}) = \frac{I_{fluo}}{M'_d} \times \frac{d^2}{d_{ref}^2} \text{ (7a)}$$

$$\text{ou }, I'_{fluo} = f_d(I_{fluo}) = \frac{I_{fluo}}{M'_d} \times \frac{d(r')^2}{d_{ref}^2} \text{ (7b)}$$

ou encore

$$I'_{fluo} = f_d(I_{fluo}) = \frac{I_{fluo}}{M'_d} \times \frac{D(r)^2}{d_{ref}^2} \text{ (7c)}$$

$M'_d$ désigne ici l'image d'éclairement normalisée, telle que décrite dans une variante du premier mode de réalisation.

En lien avec la figure 7C, on décrit à présent les principales étapes d'un procédé de correction d'une image de fluorescence en fonction de la distance d entre la source de lumière d'excitation 11 et l'objet 10, selon ce troisième mode de réalisation. Les étapes 100 et 200 sont similaires à celles décrites relativement à la figure 7A. L'étape 500 consiste à appliquer une fonction de correction de distance $f_d$, dépendant de ladite distance d, à chaque image de fluorescence $I_{fluo}$, de façon à obtenir une image de fluorescence $I'_{fluo}$ corrigée, selon l'équation (7a) , (7b) ou (7c). D'autres fonctions de correction connues peuvent également être prises en compte, en particulier une fonction de correction relative au temps d'exposition, agissant sur chaque image de façon à générer des images normalisées selon un temps d'exposition de référence $t_{ref}$, préalablement défini, par exemple 50 ms.

Ainsi, dans un quatrième mode de réalisation, la fonction de correction $f_d$ peut comporter un terme de normalisation, sous la forme d'un ratio entre le temps d'exposition t de l'image de fluorescence et le temps d'exposition de référence $t_{ref}$. Par exemple, selon ce mode de réalisation, la fonction de correction $f_d$ peut-être telle que :

$$I'_{fluo} = f_d(I_{fluo}) = \frac{I_{fluo}}{M'_d} \times \frac{d^2}{d_{ref}^2} \times \frac{t_{ref}}{t} \text{ (8a)}$$

ou :

$$I'_{fluo} = f_d(I_{fluo}) = \frac{I_{fluo}}{M_d} \times \frac{t_{ref}}{t} \text{ (8b)}$$

ou :

$$I'_{fluo} = f_d(I_{fluo}) = \frac{I_{fluo}}{M'_d} \times \frac{d^2}{d_{ref}^2} \times \frac{t_{ref}}{t} \text{ (8c)}$$

On note que les équations (8a) et (8c) utilisent l'image d'éclairement normalisée $M'_d$ tandis que l'équation (8c) utilise l'image d'éclairement non normalisée $M_d$.

Naturellement, lorsque la mesure de distance est résolue spatialement, c'est-à-dire lorsqu'on peut établir une distance d(r') entre chaque élément de surface $\delta S(r')$ conjugué d'un pixel r de l'image de fluorescence, alors :

$$I'_{fluo} = f_d(I_{fluo}) = \frac{I_{fluo}}{M'_d} \times \frac{d(r')^2}{d_{ref}^2} \times \frac{t_{ref}}{t} \quad \text{(8d)}$$

ou :

$$I'_{fluo} = f_d(I_{fluo}) = \frac{I_{fluo}}{M'_d} \times \frac{d(r')^2}{d_{ref}^2} \times \frac{t_{ref}}{t} \quad \text{(8e)}$$

ou encore

$$I'_{fluo} = f_d(I_{fluo}) = \frac{I_{fluo}}{M'_d} \times \frac{D(r)^2}{d_{ref}^2} \times \frac{t_{ref}}{t} \quad \text{(8f)}$$

On note que les équations (8a), (8c), (8d), (8e) et (8f) utilisent l'image d'éclairement normalisée $M'_d$ tandis que l'équation (8b) utilise l'image d'éclairement non normalisée $M_d$.

En lien avec la figure 7D, on décrit à présent les principales étapes d'un procédé de correction d'une image de fluorescence en fonction de la distance d entre la source de lumière d'excitation 11 et l'objet 10, selon ce deuxième mode de réalisation. Les étapes 100 et 200 sont similaires à celles décrites relativement à la figure 7A. L'étape 600 consiste à appliquer une fonction de correction de distance $f_d$, dépendant de ladite distance d, à chaque image de fluorescence $I_{fluo}$, de façon à obtenir une image de fluorescence $I'_{fluo}$ corrigée, selon l'une des équations (8a) à (8f).

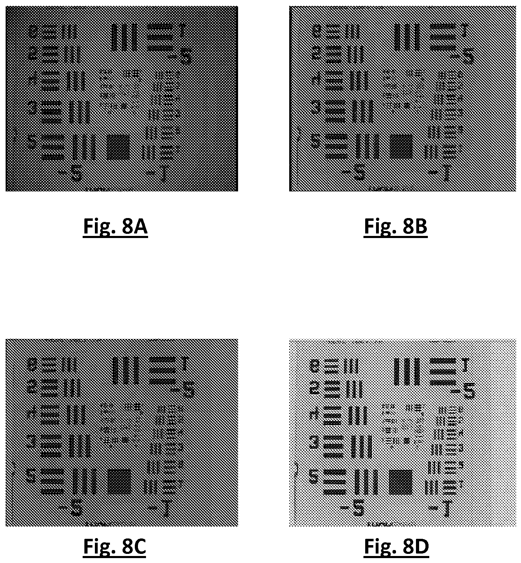

Les figures 8A à 8D, illustrent l'effet des fonctions de corrections précédemment décrites sur une image. La figure 8A a été obtenue en plaçant la sonde 1 représentée sur la figure 2 face à une mire comportant des motifs sombres formés sur un écran fluorescent tel que précédemment décrit. La mire est éclairée par un faisceau d'excitation, dans une bande spectrale d'excitation centrée sur la longueur d'onde 750 nm, et l'image de fluorescence est réalisée en collectant une lumière de fluorescence dans une bande spectrale de fluorescence s'étendant entre 800 nm et 850 nm. La sonde est placée à une distance d de 18cm ; le temps d'exposition t s'élève à 40 ms.

La figure 8B représente une image réalisée en utilisant une fonction de correction telle que décrite dans le premier mode de réalisation, en divisant, terme à terme, l'image de fluorescence $I_{fluo}$ par une image d'éclairement normalisée $M'_d$, correspondant à la distance d = 18 cm. On observe que les niveaux de gris de l'image obtenue, en dehors des motifs de la mire, sont plus homogènes. En particulier, les bordures de l'image ne sont pas assombries comme sur l'image 8A.

La figure 8C représente une image réalisée en utilisant une fonction de correction telle que décrite dans le troisième mode de réalisation, combinant la correction de l'éclairement et la compensation de la distance, la distance de référence $d_{ref}$ étant égale à 20 cm. On observe un léger assombrissement de l'image, du fait de la multiplication de chaque pixel par le scalaire $\frac{18^2}{20^2} \approx 0{,}8$.

La figure 8D représente une image réalisée en utilisant une fonction de correction telle que décrite dans le quatrième mode de réalisation, combinant la correction de l'éclairement, la compensation de la distance par rapport à une distance de référence de 20 cm, et la normalisation du temps d'exposition, le temps d'exposition de référence $t_{ref}$ étant de 83 ms. La comparaison entre les images 8C et 8D permet de visualiser l'effet de la normalisation par le temps d'exposition, avec une augmentation significative du niveau de gris des pixels les plus clairs du fait de la multiplication de chaque pixel par le scalaire $\frac{83}{40} \approx 2$.

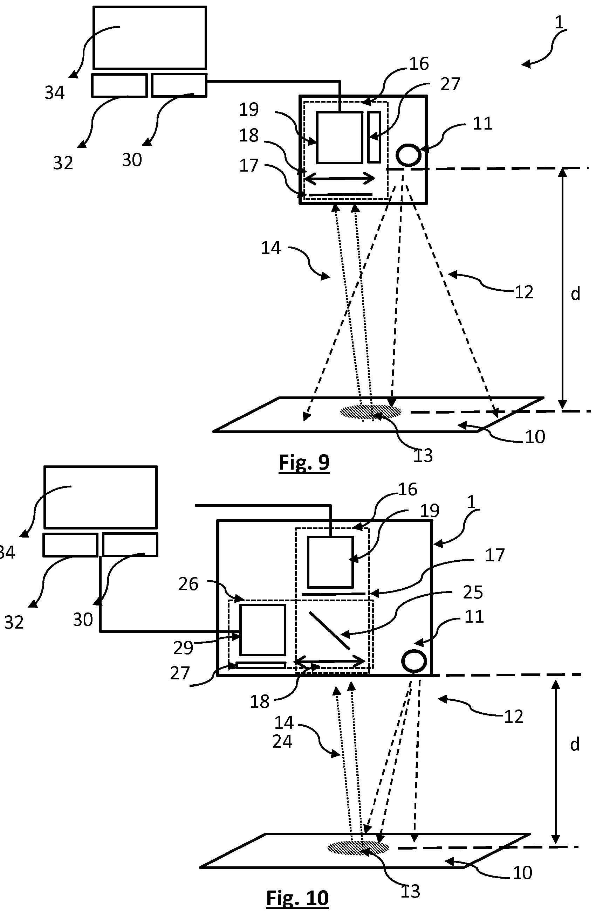

La figure 9 représente un autre mode de réalisation, dans lequel la source de lumière d'excitation est intégrée dans la sonde 1. Selon ce mode de réalisation, la mesure de distance entre la source de lumière d'excitation 11 et l'objet 10 n'est pas réalisée à l'aide d'un télémètre, mais en utilisant un système autofocus 27, apte à effectuer une mise au point automatique du capteur d'image de fluorescence 16 par rapport à l'objet 10. Ce système autofocus 27 est classique. Il effectue une mise au point sur la base d'une mesure du contraste de l'image de fluorescence $I_{fluo}$ en considérant différentes distances de mise au point. Selon une variante, le système autofocus 27 est basé sur un procédé dit à détection de phase, également connu de l'homme du métier. Le système autofocus 27 est apte à fournir une information de distance d entre la source de lumière d'excitation 11, solidaire de la sonde 1, et l'objet examiné. Il fait alors fonction de détecteur de distance.

Les inventeurs considèrent cependant qu'il est préférable de disposer d'un télémètre 20 de type optique, acoustique ou à micro-ondes, comme précédemment évoqué, car cela permet d'obtenir une mesure plus précise et plus rapide de la distance. En effet, l'image de fluorescence n'est souvent pas suffisamment contrastée pour permettre une mesure suffisamment fiable de cette distance à l'aide d'un système autofocus. De plus, une détermination automatique de la mise au point par un système autofocus peut être lente, et non compatible avec les contraintes de temps réel requises lors d'une intervention chirurgicale.

La figure 10 représente un autre mode de réalisation, dans lequel la sonde 1 comporte un séparateur 25, par exemple une lame semi-réfléchissante, apte à diriger le rayonnement optique de fluorescence 14, incident à la sonde, vers le photodétecteur matriciel 19, à travers le filtre de fluorescence 17 précédemment décrit, pour former une image de fluorescence ($I_{fluo}$). Le séparateur 25 est également apte à diriger un rayonnement optique 24 incident à la sonde 1, dans une bande spectrale visible $\lambda_{visible}$, vers un deuxième photodétecteur matriciel 29, apte à former une image en lumière visible $I_{visible}$ de l'objet examiné. Le photodétecteur matriciel 29, le séparateur 25 et l'objectif 18 forment un capteur d'image visible 26. Par bande spectrale visible, on entend une bande spectrale s'étendant dans les longueurs d'onde visibles, par exemple entre 400 nm et 700 nm.

Le processeur 30 peut alors effectuer une superposition entre l'image de fluorescente $I_{fluo}$, générée par le capteur d'image de fluorescence 16, et l'image visible de l'objet, $I_{visible}$, générée par le capteur d'image visible 26. De préférence, les axes optiques du capteur d'image visible 16 et du capteur d'image de fluorescence 26 sont confondus, de manière à faciliter une telle superposition. Dans cet exemple, l'objectif 18 est commun au capteur d'image visible 26 et au capteur d'image de fluorescence 16. Selon une variante, le capteur d'image visible comprend un objectif 28 qui lui est propre.

Le capteur d'image visible 26 peut comprendre un système de mise au point automatique, ou autofocus, 27, l'image visible $I_{visible}$ de l'objet étant alors utilisée pour le réglage automatique de la mise au point. Le système autofocus 27 est apte à fournir une information de distance d entre la source de lumière d'excitation, solidaire de la sonde 1, et l'objet examiné. Il fait alors fonction de détecteur de distance.

Précisons que l'intégration d'un capteur d'image visible 26, apte à former une image de l'objet en lumière visible $I_{visible}$, peut être prévue dans l'ensemble des modes de réalisation exposés dans cette demande.

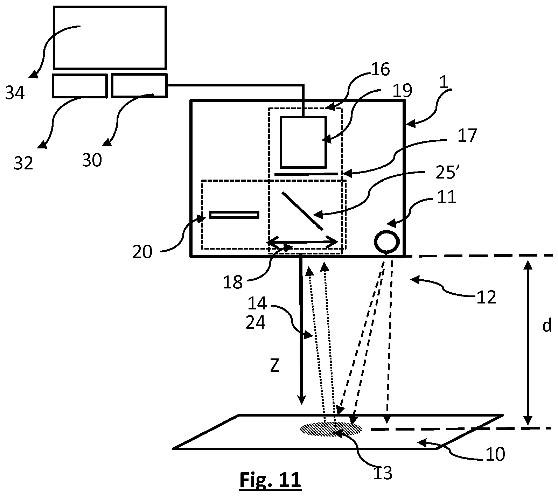

La figure 11 représente un mode de réalisation selon lequel le télémètre 20 est un télémètre optique. Par le biais d'un miroir semi-réfléchissant 25', le télémètre 20 est apte à émettre une onde lumineuse à travers le système optique 18 du capteur de fluorescence 16. Le miroir semi-réfléchissant 25' réfléchit l'onde optique 22 émise par le télémètre 20, de même que l'onde optique 22' réfléchie par l'objet. Il transmet la lumière de fluorescence 14 émise par l'objet 10 vers le photodétecteur de fluorescence 19.

De préférence, le télémètre est positionné de telle sorte que l'onde lumineuse qu'il génère est centrée par rapport audit système optique 18. Elle se propage alors vers l'objet 10 selon l'axe optique Z du capteur de fluorescence 16. Cela permet de réaliser une mesure de la distance entre la sonde 1 et le point de l'objet situé au centre de l'image acquise par le capteur 16, et cela quel que soit la distance entre la sonde et l'objet. On connaît alors précisément la zone de l'objet visée par le télémètre 20, à partir de laquelle la distance d est déterminée.

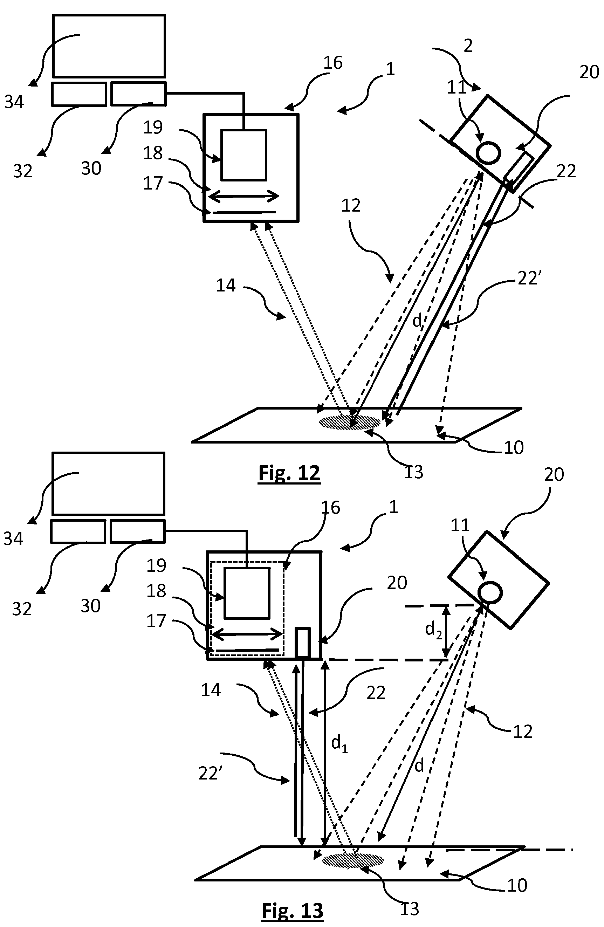

La figure 12 représente un autre mode de réalisation, dans lequel la source de lumière d'excitation 11 et le télémètre 20 sont disposés dans un module 2 indépendant de la sonde 1. Par exemple, selon ce mode de la réalisation, le module 2 est un scialytique dans lequel sont intégrés le télémètre et la source de lumière d'excitation 11. Le scialytique permet alors d'éclairer l'objet 10 non seulement dans une large bande spectrale visible, de la même façon qu'un scialytique conventionnel, mais également plus spécifiquement dans la bande spectrale d'excitation $\lambda_{ex}$ des fluorophores potentiellement présents dans l'objet. L'intégration de la source de lumière d'excitation 11 dans un scialytique permet généralement d'obtenir une répartition spatiale de l'éclairement plus homogène. Selon ce mode de réalisation, il est préférable de mettre en oeuvre une correction selon le carré de la distance, exposée en lien avec la figure 7B, complétée éventuellement par une correction selon le temps d'exposition.

La figure 13 représente un autre mode de réalisation, dans lequel la source de lumière d'excitation 11 est intégrée dans un module 2 indépendant de la sonde 1, tandis que le télémètre 20 est solidaire de la sonde 1. La position du module 2 par rapport à la sonde 1 est connue, en particulier la distance $d_2$ entre la source de lumière d'excitation 11 et le télémètre 20, et éventuellement l'orientation du module 2 par rapport à la sonde 1. Selon ce mode de réalisation, le télémètre 20 mesure la distance $d_1$ le séparant de l'objet 10, le processeur 30 étant alors apte à déterminer la distance d entre la source de lumière d'excitation 11 et l'objet 10 en fonction de la mesure de la distance $d_1$ et de la connaissance de la distance $d_2$, et éventuellement l'orientation, du module 2 par rapport à la sonde 1. Selon ce mode de réalisation, il est préférable de mettre en oeuvre une correction selon le carré de la distance, exposée en lien avec la figure 7B, complétée éventuellement par une correction selon le temps d'exposition.

Dans les modes de réalisation décrits sur les figures 1, 2, 11, 12 et 13, le télémètre 20 peut être un télémètre résolu spatialement, permettant d'obtenir une pluralité de distances d(r') entre respectivement la source d'excitation et une pluralité d'éléments de surface $\delta S(r')$ de l'objet (10), comme précédemment décrit.

La sonde 1 telle que précédemment décrite peut être mise en oeuvre dans des applications de type chirurgie ouverte, ce qui permet l'observation d'un champ opératoire. Lorsqu'elle intègre la source de lumière d'excitation 11, comme cela est représenté sur les figures 1, 8 et 9, elle peut également être utilisée dans d'autres modalités, en particulier en endoscopie ou en laparoscopie, moyennant une adaptation de ses dimensions.

Comme précisé dans les premiers paragraphes de la description détaillée, bien que les modes de réalisation décrits concernent l'imagerie de fluorescence, qui constitue la principale application visée à court terme, les principes décrits peuvent être généralisés à tout type d'image $I_{em}$ émise par l'objet, quelle que soit la bande spectrale d'émission, et en particulier une image d'un rayonnement réfléchi par l'objet ou une image d'un rayonnement rétrodiffusé par l'objet, la bande spectrale d'émission correspondant alors à tout ou partie de la bande spectrale d'excitation.

**Revendications**

1. Procédé de correction d'une image de fluorescence, comprenant les étapes suivantes :

   - illumination d'un objet (10) à l'aide d'une source de lumière d'excitation (11),
   - détection d'un rayonnement de fluorescence (14) par un capteur d'image de fluorescence (16), le rayonnement de fluorescence (14) étant émis par l'objet (10) sous l'effet de ladite illumination,
   - acquisition de ladite image de fluorescence ($I_{fluo}$) par ledit capteur d'image de fluorescence (16) à partir du rayonnement de fluorescence (14) détecté,
   - l'application d'une fonction de correction d'éclairement ($f, f_d$) à l'image de fluorescence ($I_{fluo}$), ladite fonction de correction utilisant une image d'éclairement ($M, M_d, M'_d, M_{G,d}$), représentative de la distribution spatiale d'un éclairement ($\phi$) produit, sur l'objet (10), par la source de lumière d'excitation (11),

   le procédé étant **caractérisé en ce qu'**il comporte une étape de mesure d'une distance $(d)$ entre la source d'excitation (11) et l'objet (10), la fonction de correction $(f_d)$ étant dépendante de la distance mesurée $(d)$, et utilisant une image d'éclairement ($M_d, M'_d, M_{G,d}$), associée à ladite distance $(d)$, la fonction de correction étant telle que deux images d'éclairement ($M_{d'}, M_{d''}$), respectivement associées à deux distances différentes $(d', d'')$, sont représentatives de deux distributions spatiales de l'éclairement qui sont différentes l'une de l'autre et inhomogènes.

2. Procédé de correction selon la revendication 1, la fonction de correction ($f, f_d$) comprenant un ratio, terme à terme, de l'image de fluorescence ($I_{fluo}$) par l'image d'éclairement ($M, M'_d, M_d, M_{G,d}$).

3. Procédé de correction selon l'une quelconque des revendications 1 à 2, dans lequel l'image de fluorescence étant acquise selon un temps d'exposition $(t)$, la fonction de correction est également apte à normaliser l'image de fluorescence ($I_{fluo}$) par rapport à un temps d'exposition de référence ($t_{ref}$).

4. Procédé de correction selon l'une quelconque des revendications 1 à 3, dans lequel ladite fonction de correction est également apte à corriger l'image de fluorescence ($I_{fluo}$) en fonction du carré de ladite distance mesurée $(d)$.

5. Procédé de correction selon la revendication 4, dans lequel la fonction de correction prend en compte le carré de la distance mesurée $(d)$ ainsi que le carré d'une distance de référence ($d_{ref}$).

6. Procédé de correction selon la revendication 5, comportant une étape de sélection de la distance de référence ($d_{ref}$) en fonction de l'image de fluorescence ($I_{fluo}$).

7. Procédé de correction selon l'une quelconque des revendications 5 ou 6, dans lequel la fonction de correction ($f_d$) effectue un produit de l'image de fluorescence ($I_{fluo}$) par un ratio ( $\frac{d^2}{d_{ref}^2}$ ) entre le carré de ladite distance mesurée et le carré de ladite distance de référence.

8. Procédé de correction selon l'une quelconque des revendications 1 à 7, dans lequel la distance $(d)$ entre la source de lumière d'excitation (11) et l'objet (10) est mesurée au moyen d'un télémètre (20) apte à émettre une onde (22) en direction de l'objet (10) et à détecter une onde (22') réfléchie par ledit objet, en fonction de laquelle ladite distance $(d)$ est mesurée.

9. Procédé de correction selon l'une quelconque des revendications 1 à 8, dans lequel la distance $(d)$ entre la source de lumière d'excitation et l'objet (10) est mesurée par un système autofocus (27), apte à établir automatiquement une distance de mise au point du capteur d'image de fluorescence (16) par rapport à l'objet (10).

**10.** Procédé de correction selon l'une quelconque des revendications précédentes, comportant également une étape d'acquisition d'une image visible ($I_{visible}$) de l'objet (10) à l'aide d'un capteur d'image visible (26).

**11.** Dispositif d'acquisition (1) d'une image de fluorescence comportant :

- une source de lumière d'excitation (11), apte à illuminer un objet (10) dans une bande spectrale d'excitation ($\lambda_{ex}$),
- un capteur d'image de fluorescence (16), apte à collecter un rayonnement de fluorescence (14) émis par l'objet (10), dans une bande spectrale de fluorescence ($\lambda_{fluo}$) sous l'effet de l'illumination par la source de lumière d'excitation, le capteur d'image de fluorescence étant apte à acquérir une image de fluorescence ($I_{fluo}$) à partir du rayonnement de fluorescence collecté,
- un télémètre (20) pour mesurer une distance ($d$) entre la source de lumière d'excitation (11) et l'objet (10), le télémètre (20) étant apte à émettre une onde (22) en direction de l'objet (10) et à détecter une onde optique (22') réfléchie par ledit objet, en fonction de laquelle ladite distance ($d$) est mesurée,

le dispositif d'acquisition étant **caractérisé en ce qu'**il comporte également un processeur, apte à mettre en oeuvre le procédé de correction de l'image de fluorescence ($I_{fluo}$) objet de l'une quelconque des revendications 1 à 10.

**12.** Dispositif d'acquisition d'une image de fluorescence selon la revendication 11, dans lequel le capteur d'image de fluorescence est centré autour d'un axe optique (Z), le télémètre (20) étant apte à émettre une onde optique selon ledit axe optique.

**13.** Dispositif d'acquisition d'une image de fluorescence selon l'une quelconque des revendications 11 ou 12, **caractérisé en ce que** le capteur de fluorescence comporte un système optique de focalisation (18), le télémètre (20) étant configuré pour émettre une onde optique, en direction de l'objet, centrée selon ce système optique.

**14.** Dispositif d'acquisition d'une image de fluorescence selon l'une quelconque des revendications 11 à 13, **caractérisé en ce qu'**il comporte également un sélecteur (15) d'une distance de référence *($d_{ref}$)*, pour mémoriser ladite distance de référence dans une mémoire (32).

**Patentansprüche**

**1.** Verfahren zur Korrektur eines Fluoreszenzbilds, das die folgenden Schritte enthält:

- Beleuchtung eines Objekts (10) mit Hilfe einer Anregungslichtquelle (11),
- Erkennung einer Fluoreszenzstrahlung (14) durch einen Fluoreszenzbild-Sensor (16), wobei die Fluoreszenzstrahlung (14) unter der Wirkung der Beleuchtung vom Objekt (10) emittiert wird,
- Erfassung des Fluoreszenzbilds ($I_{fluo}$) durch den Fluoreszenzbild-Sensor (16) ausgehend von der erkannten Fluoreszenzstrahlung (14),
- Anwendung einer Beleuchtungsstärke-Korrekturfunktion (f, $f_d$) an das Fluoreszenzbild ($I_{fluo}$), wobei die Korrekturfunktion ein Beleuchtungsstärkebild (M, $M_d$, M'$_d$, $M_{G,d}$) verwendet, das für die räumliche Verteilung einer Beleuchtungsstärke ($\phi$) repräsentativ ist, die auf dem Objekt (10) durch die Anregungslichtquelle (11) erzeugt wird,

wobei das Verfahren **dadurch gekennzeichnet ist, dass** es einen Schritt der Messung eines Abstands (d) zwischen der Anregungsquelle (11) und dem Objekt (10) aufweist, wobei die Korrekturfunktion ($f_d$) vom gemessenen Abstand (d) abhängt, und ein dem Abstand (d) zugeordnetes Beleuchtungsstärkebild ($M_d$, M'$_d$, $M_{G,d}$) verwendet, wobei die Korrekturfunktion so ist, dass zwei Beleuchtungsstärkebilder ($M_{d'}$, $M_{d"}$), die jeweils zwei unterschiedlichen Abständen (d', d") zugeordnet sind, für zwei räumliche Verteilungen der Beleuchtungsstärke repräsentativ sind, die sich voneinander unterscheiden und nicht homogen sind.

**2.** Korrekturverfahren nach Anspruch 1, wobei die Korrekturfunktion (f,$f_d$) ein Verhältnis, Term für Term, des Fluoreszenzbilds ($I_{fluo}$) durch das Beleuchtungsstärkebild (M, M'$_d$, $M_d$, $M_{G,d}$) enthält.

**3.** Korrekturverfahren nach einem der Ansprüche 1 bis 2, wobei, da das Fluoreszenzbild gemäß einer Belichtungszeit (t) erfasst wird, die Korrekturfunktion ebenfalls fähig ist, das Fluoreszenzbild ($I_{fluo}$) bezüglich einer Bezugsbelichtungszeit (tref) zu normieren.

**4.** Korrekturverfahren nach einem der Ansprüche 1 bis 3, wobei die Korrekturfunktion ebenfalls fähig ist, das Fluoreszenzbild ($I_{fluo}$) abhängig vom Quadrat des gemessenen Abstands (d) zu korrigieren.

**5.** Korrekturverfahren nach Anspruch 4, wobei die Korrekturfunktion das Quadrat des gemessenen Abstands (d) sowie das Quadrat eines Bezugsabstands ($d_{ref}$) berücksichtigt.

**6.** Korrekturverfahren nach Anspruch 5, das einen Schritt der Auswahl des Bezugsabstands ($d_{ref}$) abhängig vom Fluoreszenzbild ($I_{fluo}$) aufweist.

**7.** Korrekturverfahren nach einem der Ansprüche 5 oder 6, wobei die Korrekturfunktion ($f_d$) ein Produkt aus dem Fluoreszenzbild ($I_{fluo}$) und einem Verhältnis ( $\frac{d^2}{d_{ref}^2}$ ) zwischen dem Quadrat des gemessenen Abstands und dem Quadrat des Bezugsabstands ausführt.

**8.** Korrekturverfahren nach einem der Ansprüche 1 bis 7, wobei der Abstand (d) zwischen der Anregungslichtquelle (11) und dem Objekt (10) mittels eines Entfernungsmessers (20) gemessen wird, der fähig ist, eine Welle (22) in Richtung des Objekts (10) zu emittieren und eine vom Objekt reflektierte Welle (22') zu erkennen, abhängig von der der Abstand (d) gemessen wird.

**9.** Korrekturverfahren nach einem der Ansprüche 1 bis 8, wobei der Abstand (d) zwischen der Anregungslichtquelle und dem Objekt (10) von einem Autofokus-System (27) gemessen wird, das fähig ist, automatisch eine Fokusentfernung des Fluoreszenzbild-Sensors (16) bezüglich des Objekts (10) zu erstellen.

**10.** Korrekturverfahren nach einem der vorhergehenden Ansprüche, das ebenfalls einen Schritt der Erfassung eines sichtbaren Bilds ($I_{visible}$) des Objekts (10) mit Hilfe eines Sensors eines sichtbaren Bilds (26) aufweist.

**11.** Erfassungsvorrichtung (1) eines Fluoreszenzbilds, die aufweist:

- eine Anregungslichtquelle (11), die fähig ist, ein Objekt (10) in einem Anregungsspektralband ($\lambda_{ex}$) zu beleuchten,
- einen Fluoreszenzbild-Sensor (16), der fähig ist, eine vom Objekt (10) emittierte Fluoreszenzstrahlung (14) in einem Fluoreszenzspektralband ($\lambda_{fluo}$) unter der Wirkung der Beleuchtung durch die Anregungslichtquelle zu sammeln, wobei der Fluoreszenzbild-Sensor fähig ist, ausgehend von der gesammelten Fluoreszenzstrahlung ein Fluoreszenzbild ($I_{fluo}$) zu erfassen,
- ein Entfernungsmesser (20), um einen Abstand (d) zwischen der Anregungslichtquelle (11) und dem Objekt (10) zu messen, wobei der Entfernungsmesser (20) fähig ist, eine Welle (22) in Richtung des Objekts (10) zu emittieren und eine vom Objekt reflektierte optische Welle (22') zu erkennen, abhängig von der der Abstand (d) gemessen wird,

wobei die Erfassungsvorrichtung **dadurch gekennzeichnet ist, dass** sie ebenfalls einen Prozessor aufweist, der fähig ist, das Korrekturverfahren des Fluoreszenzbilds ($I_{fluo}$) durchzuführen, das Gegenstand eines der Ansprüche 1 bis 10 ist.

**12.** Erfassungsvorrichtung eines Fluoreszenzbilds nach Anspruch 11, wobei der Fluoreszenzbild-Sensor um eine optische Achse (Z) zentriert ist, wobei der Entfernungsmesser (20) fähig ist, eine optische Welle gemäß der optischen Achse zu emittieren.

**13.** Erfassungsvorrichtung eines Fluoreszenzbilds nach einem der Ansprüche 11 oder 12, **dadurch gekennzeichnet, dass** der Fluoreszenzsensor ein optisches Fokussiersystem (18) aufweist, wobei der Entfernungsmesser (20) konfiguriert ist, eine optische Welle in Richtung des Objekts zu emittieren, die gemäß diesem optischen System zentriert ist.

**14.** Erfassungsvorrichtung eines Fluoreszenzbilds nach einem der Ansprüche 11 bis 13, **dadurch gekennzeichnet, dass** sie ebenfalls einen Wählschalter (15) eines Bezugsabstands ($d_{ref}$) aufweist, um den Bezugsabstand in einem Speicher (32) zu speichern.

**Claims**

1. Method for correcting a fluorescence image, comprising the following steps:

   - illuminating an object (10) using an excitation light source (11);
   - detecting fluorescence radiation (14) with a fluorescence-image sensor (16), the fluorescence radiation (14) being emitted by the object (10) under the effect of said illumination;
   - acquiring said fluorescence image ($I_{fluo}$) with said fluorescence-image sensor (16) on the basis of the detected fluorescence radiation (14); and
   - applying an illuminance correction function ($f$, $f_d$) to the fluorescence image ($I_{fluo}$), said correction function using an illuminance image ($M$, $M_d$, $M'_d$, $M_{G,d}$) representative of the spatial distribution of an illuminance ($\Phi$) produced, on the object (10), by the excitation light source (11),

   the method being **characterized in that** it includes a step of measuring a distance ($d$) between the excitation source (11) and the object (10), the correction function ($f_d$) being dependent on the measured distance (d), and using an illuminance image ($M_d$, $M'_d$, $M_{G,d}$) associated with said distance ($d$), the correction function being such that two illuminance images ($M_{d'}$, $M_{d''}$) respectively associated with two different distances ($d'$, $d''$) are representative of two spatial distributions of illuminance that are different from each other and non-uniform.

2. Correcting method according to Claim 1, the correction function ($f$, $f_d$) comprising a term-by-term ratio of the fluorescence image ($I_{fluo}$) to the illuminance image ($M$, $M'_d$, $M_d$, $M_{G,d}$).

3. Correcting method according to either one of Claims 1 and 2, wherein the fluorescence image being acquired in an exposure time ($t$), the correction function is also able to normalize the fluorescence image ($I_{fluo}$) with respect to a reference exposure time ($t_{ref}$).

4. Correcting method according to any one of Claims 1 to 3, wherein said correction function is also able to correct the fluorescence image ($I_{fluo}$) depending on the square of said measured distance ($d$).

5. Correcting method according to Claim 4, wherein the correction function takes into account the square of the measured distance ($d$) and the square of a reference distance ($d_{ref}$).

6. Correcting method according to Claim 5, including a step of selecting the reference distance ($d_{ref}$) depending on the fluorescence image ($I_{fluo}$).

7. Correcting method according to either one of Claims 5 and 6, wherein the correction function ($f_d$) applies a product

$$\frac{d^2}{d_{ref}^2}$$

   of the fluorescence image ($I_{fluo}$) multiplied by a ratio ( $\frac{d^2}{d_{ref}^2}$ ) between the square of said measured distance and the square of said reference distance.

8. Correcting method according to any one of Claims 1 to 7, wherein the distance ($d$) between the excitation light source (11) and the object (10) is measured by means of a rangefinder (20) that is able to emit a wave (22) in the direction of the object (10) and to detect a wave (22') reflected by said object, depending on which wave said distance ($d$) is measured.

9. Correcting method according to any one of Claims 1 to 8, wherein the distance ($d$) between the excitation light source and the object (10) is measured by an autofocus system (27) that is able to automatically establish a focus distance of the fluorescence-image sensor (16) with respect to the object (10).

10. Correcting method according to any one of the preceding claims, also including a step of acquiring a visible image ($I_{visible}$) of the object (10) using a visible-image sensor (26).

11. Device (1) for acquiring a fluorescence image, including:

    - an excitation light source (11) able to illuminate an object (10) in an excitation spectral band ($\lambda_{ex}$);
    - a fluorescence-image sensor (16) able to collect fluorescence radiation (14) emitted by the object (10), in a

fluorescence spectral band ($\lambda_{fluo}$), under the effect of the illumination by the excitation light source, the fluorescence-image sensor being able to acquire a fluorescence image ($I_{fluo}$) on the basis of the collected fluorescence radiation; and

- a rangefinder (20) for measuring a distance (*d*) between the excitation light source (11) and the object (10), the rangefinder (20) being able to emit a wave (22) in the direction of the object (10) and to detect an optical wave (22') reflected by said object, depending on which wave said distance (*d*) is measured,

the acquiring device being **characterized in that** it also includes a processor able to implement the method for correcting the fluorescence image ($I_{fluo}$) that is the subject of any one of Claims 1 to 10.

12. Device for acquiring a fluorescence image according to Claim 11, wherein the fluorescence-image sensor is centered on an optical axis (Z), the rangefinder (20) being able to emit an optical wave along said optical axis.

13. Device for acquiring a fluorescence image according to either one of Claims 11 and 12, **characterized in that** the fluorescence sensor includes a focusing optical system (18), the rangefinder (20) being configured to emit an optical wave in the direction of the object, said wave being centered using this optical system.

14. Device for acquiring a fluorescence image according to any one of Claims 11 to 13, **characterized in that** it also includes a selector (15) of a reference distance ($d_{ref}$), in order to store said reference distance in a memory (32).

**Fig. 1**

**Fig. 2**

**Fig. 3A**

**Fig. 3B**

**Fig. 4A**

**Fig. 4B**

**Fig. 5A**

**Fig. 5B**

**Fig. 6A**

**Fig. 6B**

**Fig. 6C**

100

$I_{fluo}$

200

$d$

$M_d, M'_d, M$

300

$I'_{fluo} = f_d(I_{fluo})$

**Fig. 7A**

100

$I_{fluo}$

200

$d, D, d(r')$

$d_{ref}$

400

$I'_{fluo} = f_d(I_{fluo})$

**Fig. 7B**

100

$I_{fluo}$

200

$d, D, d(r')$

$M'_d, d_{ref}$

500

$I'_{fluo} = f_d(I_{fluo})$

**Fig. 7C**

100

$I_{fluo}$

200

$d, D, d(r')$

$M_d, M'_d,$
$d_{ref}, t_{ref}$

600

$I'_{fluo} = f_d(I_{fluo})$

**Fig. 7D**

Fig. 8A

Fig. 8B

Fig. 8C

Fig. 8D

**Fig. 9**

**Fig. 10**

**Fig. 11**

**Fig. 12**

**Fig. 13**

**EP 3 295 153 B1**

## RÉFÉRENCES CITÉES DANS LA DESCRIPTION

**Documents brevets cités dans la description**

- US 8606350 B
- EP 1829473 A
- EP 2505988 A
- US 2013113907 A
- US 5749830 A
- US 6280378 B
- EP 2412296 A